# EUROPEAN PATENT SPECIFICATION

(11) **EP 4 472 708 B1**
(45) Date of publication and mention of the grant of the patent: **25.11.2026**
(21) Application number: 23708295.3
(22) Date of filing: 02.02.2023
(51) Int. Cl.: A61M 16/20, A61M 16/08, A61M 16/04, A61M 16/16, A61M 16/00

(54) **DEVICE FOR MAINTAINING PEEP IN A PATIENT, AND MECHANICAL VENTILATION CIRCUIT COMPRISING SAID DEVICE**
VORRICHTUNG ZUR PEEP-AUFRECHTERHALTUNG BEI EINEM PATIENTEN UND MECHANISCHER BEATMUNGSKREISLAUF MIT DIESER VORRICHTUNG
DISPOSITIF DE MAINTIEN DE LA PRESSION POSITIVE RESPIRATOIRE CHEZ UN PATIENT, ET CIRCUIT DE VENTILATION MECANIQUE COMPRENANT CE DISPOSITIF

(30) Priority: 03.02.2022 IT 202200001940
(43) Date of publication of application: 11.12.2024
(73) Proprietor: Universita' Degli Studi di Firenze, 50121 Firenze (IT); Azienda Ospedaliero-Universitaria Careggi, 50134 Firenze (IT)
(72) Inventor: BARDACCI, Yari, 50144 Firenze (IT); BOCCHI, Leonardo, 59016 Poggio A Caiano (PO) (IT); CASATI, Lorenzo, 50022 Greve In Chianti (FI) (IT); BALDASSINI, Samuele, 50142 Firenze (IT); BAMBI, Stefano, 50134 Firenze (IT); RASERO, Laura, 50019 Sesto Fiorentino (FI) (IT)
(74) Representative: Petraz, Davide Luigi
(86) International application number: PCT/IT2023/050027
(87) International publication number: WO 2023/148794

(56) References cited:
- EP-B1- 1 491 227
- EP-B1- 1 897 578
- US-A1- 2015 297 851
- US-A1- 2021 299 396
- US-B2- 8 161 972
- US-B2- 8 439 037

## Description

### FIELD OF THE INVENTION

The present invention concerns a device for maintaining Positive End-Expiratory Pressure (PEEP) in patients with a definitive artificial airway and connected to a positive pressure pulmonary ventilator. The device is included inside the respiratory circuit of the mechanical ventilator. In the context of the present application, by PEEP it is meant the positive end-expiratory pressure guaranteed by the mechanical ventilator during the artificial ventilation of patients. More specifically, the device integrates a sensor, a control system and a safety valve, and can be installed in mechanical ventilation circuits for respiratory assistance to patients with a definitive artificial airway (tracheal tube or tracheostomy cannula).

### BACKGROUND OF THE INVENTION

Mechanical ventilation replaces or supports the activity of the inspiratory muscles to ensure adequate volume and gas exchange to the lungs. It is a treatment that is applied to patients defined as critical, with compromised autonomous ventilation or for whom it is necessary to control the respiratory function to obtain specific therapeutic results. Mechanical ventilation is implemented through an artificial respirator, also called mechanical ventilator, which replaces or supports the autonomous respiratory activity of the patient.

Mechanical ventilation can be of the non-invasive type, if interfaces are used that do not invade the patient's airways (nasal, oronasal or full-face masks, helmets, nasal cannulas) or of the invasive type, with the aid of a supraglottic or definitive artificial airway (endotracheal tube or tracheostomy cannula). These interfaces and artificial airways are connected to a circuit to supply a gas consisting of an oxygen-air mixture.

The present invention proposes to solve a specific problem concerning invasive mechanical ventilation with a permanent airway.

More specifically, the problem is that both programmed and accidental disconnections of the tracheal tube or tracheostomy cannula from the mechanical ventilation circuit can occur. Programmed disconnection can take place in the event of routine procedures being performed (e.g., endotracheal aspiration using the open technique, replacement of the tubes of the circuit itself, or of the humidifier filters, and other less frequent procedures, such as changing the ventilator or transfer from bed to stretcher and vice versa). Accidental disconnection, on the other hand, can occur during numerous procedures or assistance activities carried out on the patient (e.g., hygiene treatments, repositioning or movements/transfers), or caused by the autonomous mobilization of the patient himself. All of these occurrences are not preventable.

Disconnection from the gas supply circuit is a significant problem because when this occurs, there is a failure of PEEP inside the lungs of the patient undergoing mechanical ventilation, causing possible complications. In cases where the lungs are affected by severe respiratory insufficiency, such as in Acute Respiratory Distress Syndrome (ARDS), we witness the phenomenon of alveolar collapse in the dependent zones of the lungs due to edema and the increase in the weight of the imbibed pulmonary parenchyma which exerts a compression of the alveoli downstream, due to an increase in the force of gravity. PEEP allows to reopen the collapsed alveoli and to keep them recruited and available for gas exchange during all the phases of the respiratory cycle. When disconnections from the ventilator occur in these types of patients, particularly if accidentally, the alveolar derecruitment due to the sudden loss of PEEP can cause a major impact on arterial oxygenation levels, with a severe worsening of the patient's clinical conditions, including the possibility, albeit rare, of death. To date, in order to contain the phenomenon, some manual systems are used (e.g. atraumatic forceps or clamps for clamping tubes), which furthermore have not yet been definitively studied in literature in terms of real effectiveness, to prevent the loss of PEEP only during disconnections programmed by the operators, and exclusively applicable on patients with a tracheal tube, since they cannot be used on patients using tracheostomy cannulas, considering the absence of sufficient clamping space and the type of material of the non-compressible cannulas.

The purpose of the invention is therefore to prevent the fall of PEEP, and the complications deriving therefrom, in patients with an endotracheal or tracheostomy tube in invasive mechanical ventilation.

Various devices are known, all based on a valve, to be inserted in mechanical ventilation circuits. The indications and characteristics of use are summarized below.

WO-A1-2021/034172: valve to be used in cases of cardiac arrest during cardiopulmonary resuscitation maneuvers, to promote the increase in airflow in the inspiratory and expiratory phases, since it is affected by the pressures exerted on the thorax during external cardiac massage. In fact, the flow is stopped during compressions of the thorax and resumed during the expiratory phase, to prevent the development of high intrathoracic pressures and at the same time to promote ventilation also during cardiopulmonary resuscitation.

US-B2-8161972: valve which, in the expiratory phase, since it is positioned in proximity to the artificial airway (more specifically, only tracheostomy cannulas) allows the passage of a part of the air exhaled by the vocal cords, effectively promoting the patient's phonation. This system works as a semi-closed system (that is, closed on inspiration and open on expiration), therefore with a reduction in the positive end-expiratory pressures during the phonation phase of the patient.

US-A1-2015/0297851: system based on a valve, and which provides a gas injection line integrated with the tracheostomy cannula to allow the phonation of the patient in alternate cycles during mechanical ventilation. The valve described in this document opens with relatively low pressures, while it closes with relatively high pressures, all intended to promote the phonation activity during the positive pressure mechanical ventilation of the patient. This system has an operation similar to that of the system in US-B2-8161972.

US-A1-2014/0261442: "continuous" control system of the inflation pressure of the cuff (anchor balloon in the tracheal lumen) of the artificial airways (orotracheal tube or a tracheostomy cannula), during positive pressure mechanical ventilation. The goal is to prevent micro-inhalation of the patient's gastric contents and prevent air leaks during inspiration by maintaining perfusion pressures of the tracheal mucosa below the hypoperfusion threshold. This system in no way provides control over pressure loss through the main lumen of the artificial airway and therefore does not prevent loss of PEEP.

EP-B1-1897578 describes a system configured to apply the so-called flow interruption technique, which allows to measure the fundamental parameters of respiratory mechanics based on the registration of tracheal pressure and esophageal pressure, and is based on the sudden interruption of the inspiratory or expiratory flow and on maintaining that interruption for a few seconds. This system is based on a valve mechanism, but correlated to a pressure sensor with the sole purpose of monitoring the pressures developed by the patient inside the circuit.

US-B2-8439037 concerns a system composed of an expiratory valve for bi-tube ventilation circuits assembled with a filter and integrated flow sensor. The advantages of this system are linked to the ability to manage the exhalation flows, filtering the gases from the particulate and collecting any possible condensation.

US 2021/220604 A1 also discloses a system for maintaining PEEP for mechanical ventilation circuits.

None of the above known solutions is able to perform the functions of the proposed device, in fact:
- they are not able to quickly recognize the accidental or intentional disconnection of the ventilation circuit, since they lack a pressure sensor located in a suitable position to perform this function, nor do they have a circuit dedicated to recognizing the particular pressure variation;
- they do not have a documented activation speed, if there is a detachment of the ventilation circuit from the definitive airway, so as to be able to interrupt the expiratory flow in a sufficiently short time to allow the positive end-expiratory pressure (PEEP) to be maintained;
- they are unable to reduce the risk of serious clinical complications;
- they do not find the particular location which on the contrary is provided for our device, which is located between the ventilation circuit and the artificial airway; this is essential to ensure, in the event of disconnection of the device from the artificial airway, both the minimization of the dead space (space in which the inspired air passes without undergoing gaseous exchanges), and also the promotion of the occlusion as close as possible to the artificial airway.

To achieve this aim, the present disclosure consists of a device which allows PEEP to be maintained by acting automatically and rapidly, thanks to the ability of the pressure sensors to perceive even the slightest variation thereof. At the same time, the device allows to close the valve mechanism integrated therewith.

The guaranteed rapid capture of the pressure drop inside the ventilation circuit, and the subsequent closure of the system, protects against the loss of PEEP which would otherwise occur in a few seconds.

Another purpose of the present invention is to provide a mechanical ventilation circuit equipped with the above device.

The Applicant has devised, tested and embodied the present invention to overcome the shortcomings of the state of the art and to obtain these and other purposes and advantages.

### SUMMARY OF THE INVENTION

The present invention is set forth and characterized in the independent claim. The dependent claims describe other characteristics of the present invention or variants to the main inventive idea.

In accordance with the above purposes, and to resolve the technical problem disclosed above in a new and original way, also achieving considerable advantages compared to the state of the prior art, a device has been devised for maintaining PEEP in a patient. Such device comprises a safety valve (which in turn comprises: a body which conforms an internal duct, a closure member for closing such internal duct and which can be displaced between an opening position of the internal duct and a closing position of the internal duct), at least one pressure sensor configured to measure the pressure inside the internal duct and a controller connected to the at least one pressure sensor and to the closure member and configured to command the closure member on the basis of signals received from at least the pressure sensor. In particular, the controller is configured to command the displacement of the closure member to the closing position of the internal duct when the at least one pressure sensor detects a decrease in the pressure, preferably a drop in pressure below a predefined threshold value. This threshold value is preferably greater than or equal to the positive end-expiratory pressure.

According to some embodiments, the body of the valve comprises a first end configured to connect to a mechanical ventilation circuit and a second end configured to connect directly to an artificial airway applicable to a patient (endotracheal tube or tracheostomy cannula). By the term "connect directly" it is meant that the second end of the valve body is configured to connect to an artificial airway without the interposition of any other element of the ventilation circuit. This makes the device configured to be connected directly to a patient's artificial airway.

In accordance with different embodiments, the first end of the body is configured to connect to the rest of a ventilation circuit, in particular to a connection tube ("Y of the circuit") connected to an inspiratory branch and to an expiratory branch of the ventilation circuit. The circuit, with the inspiratory branch and the expiratory branch, is connected to a pulmonary ventilator.

According to one aspect, there is provided a mechanical ventilation circuit equipped with a device for maintaining PEEP as described above.

In particular, the mechanical ventilation circuit comprises a pulmonary ventilator, an inspiratory branch formed by one or more gas delivery tubes and on which there is disposed an active humidifier fed with bi-distilled sterile water, an expiratory branch, a Y-shaped connector which connects both the inspiratory branch and also the expiratory branch to a corrugated connection tube (catheter mount) directly connected to an artificial airway (endotracheal tube or tracheostomy cannula).

The ventilation circuit comprises a device for guaranteeing and maintaining PEEP, interposed between the connection tube (catheter mount) and the artificial airway.

Preferably, the device, in order to guarantee and maintain PEEP, preventing it from dropping (loss), is connected directly to the artificial airway. More preferably, the device for maintaining PEEP is also connected directly to the connection tube (catheter mount).

The device as above can be useful to:
- implement a "safety" procedure to maintain PEEP in a patient which provides the steps as above in a mechanical ventilation circuit, connected directly to an artificial airway;
- monitor the pressure inside the internal duct of the valve device by means of at least one pressure sensor;
- command, by means of the controller, the displacement of the closure member of the valve into the closing position of the internal duct when the at least one pressure sensor detects a decrease in pressure, preferably a drop in pressure below a predefined threshold value.

### DESCRIPTION OF THE DRAWINGS

These and other aspects, characteristics and advantages of the present disclosure will become apparent from the following description of some embodiments, given as a non-restrictive example with reference to the attached drawings wherein:
- fig. 1 is a three-dimensional view of a safety valve in accordance with some embodiments described here;
- fig. 2 is a three-dimensional view of a first module of the safety valve of fig. 1;
- fig. 3 is a three-dimensional view of a second module of the safety valve of fig. 1;
- fig. 4 is a three-dimensional view of a third module of the safety valve of fig. 1;
- fig. 5 is a three-dimensional view of a closure member located inside the module of fig. 4;
- fig. 6 is a three-dimensional view of a safety valve in accordance with some embodiments described here, in which the first and second modules are identical with respect to the embodiment shown in figs. 1-4;
- figs. 7A and 7B are three-dimensional views of a third module of the safety valve of fig. 6, according to two different angles;
- fig. 8 is a three-dimensional view of a closure member placed in the module of fig. 7A and 7B; and
- fig. 9 is a schematic view of a mechanical ventilation circuit equipped with a valve according to the present disclosure.

It is to be clarified that in the present description the phraseology and terminology used, such as for example the terms horizontal, vertical, front, rear, high, low, internal and external, with their declinations, have the sole function of better illustrating the present invention with reference to the attached drawings and must not be in any way used to limit the scope of the invention itself, or the field of protection defined by the attached claims.

Furthermore, the people of skill in the art will recognize that certain sizes or characteristics in the drawings may have been enlarged, deformed, or shown in an unconventional or non-proportional way in order to provide a version of the present invention that is easier to understand. When sizes and/or values are specified in the following description, the sizes and/or values are provided for illustrative purposes only and must not be construed as limiting the scope of protection of the present invention, unless such sizes and/or values are present in the attached claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS

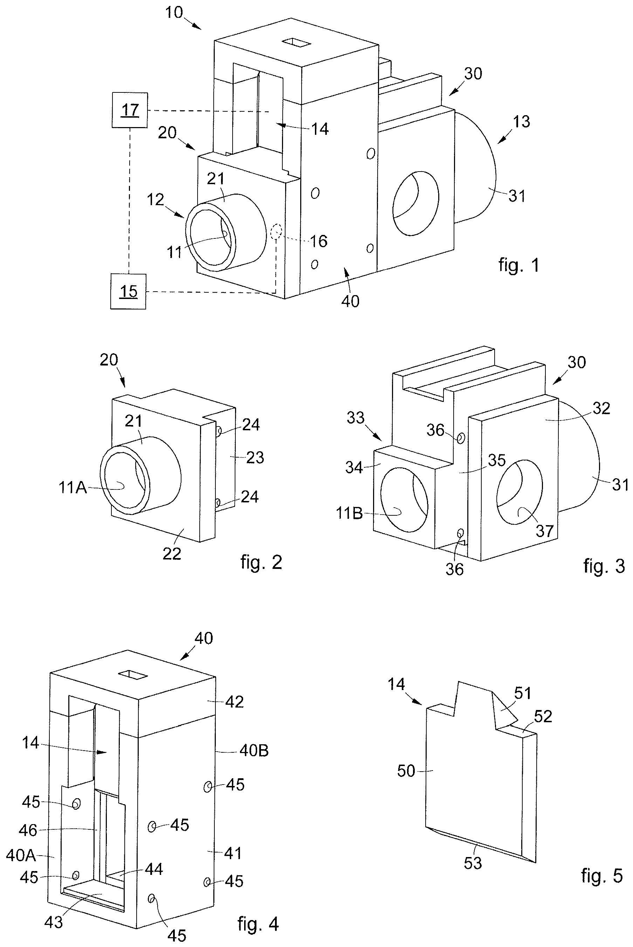

With reference to fig. 1, a device 10 for maintaining PEEP according to the present disclosure comprises a valve which in turn comprises a body inside which there is defined an internal duct 11, which passes through it from side to side.

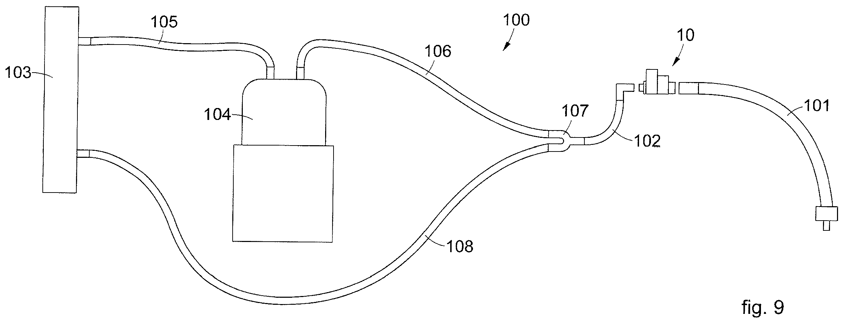

In particular, the internal duct 11 extends between a first end 12 of the body, configured to connect to a mechanical ventilation circuit 100, and a second end 13 of the body, configured to connect to an artificial airway 101 applied to a patient (fig. 9). The artificial airway 101 can be an endotracheal tube or a tracheostomy cannula. Both ends 12, 13 are configured as connectors to be connected hermetically and directly, respectively, to a connection tube 102, for example a corrugated tube or an elbow connector, of the mechanical ventilation circuit 100, and to the artificial airway 101.

A closure member 14 is provided inside the body between the first and the second end 12, 13, and positioned in such a way as to operate the hermetic closure of the internal duct 11, when necessary, in particular in the event of disconnection of the artificial airway 101 from the mechanical ventilation circuit 100.

The closure member 14 is able to be displaced between an opening position of the internal duct 11, in which the closure member 14 does not close the internal duct 11, and a closing position of the internal duct 11, in which the closure member hermetically closes the internal duct 11.

The displacement of the closure member 14 is commanded by a controller 15 connected to it, which receives signals from one or more pressure sensors 16 installed in such a way as to detect the pressure inside the internal duct 11. In this way, it is possible operate the hermetic closure of the internal duct 11 automatically, on the basis of the pressure detected inside the internal duct 11, in particular if the one or more pressure sensors 16 detect a drop in pressure, for example due to a disconnection of the artificial airway 101 from the mechanical ventilation circuit 100. It can be provided that the controller 15 is configured to command the closure of the internal duct 11 when a drop in pressure below a predefined threshold value is detected. This allows to maintain the positive end-expiratory pressure (PEEP) inside the patient's lungs even in the event of accidental disconnection of the artificial airway 101 from the rest of the mechanical ventilation circuit 100.

Preferably, the controller 15 is connected to the closure member 14 through an actuator 17 which automatically actuates the displacement of the closure member from its opening position to its closing position, and vice versa.

In other embodiments, not shown, the displacement of the closure member 14 both from the opening position to the closing position, and vice versa, is carried out manually by the operator, by acting on suitable mechanical members such as pins, levers or others. The manual drive of the closure member 14 advantageously allows to selectively disconnect the artificial airway 101 from the rest of the mechanical ventilation circuit 100 on the basis of programmed needs. For example, this can be useful either in order to more effectively perform some assistance maneuvers on the patient, or in event the artificial airway 101 inserted in the patient consists of an orotracheal tube or a tracheostomy cannula with an integrated metal structure, in both of which it is not possible to apply clamping devices to restrict the passage section of the artificial airway.

With reference to the examples described here with reference to the attached drawings, the valve of the device 10 is of the modular type, whereby its body is divided into three modules.

Fig. 2 shows a first module 20 which contains a first connector 21 which forms the first end 12 of the body, this first connector 21 being configured to connect directly to the mechanical ventilation circuit 100.

The first module 20 comprises a substantially square-shaped plate 22 and includes, in correspondence with a first surface, the first connector 21, and in correspondence with the opposite surface, a connection member 23 configured to connect to another module of the valve body, as will be explained below. The connection member 23 can be in the shape of a parallelepiped with a square section and equipped with respective connection elements 24, for example shaped as holes configured to house rivets or screws, as shown in fig. 2.

Advantageously, the first connector 21 and the connection member 23 are substantially coaxial, so that a first part 11A of the internal duct 11, preferably rectilinear, can be formed in them.

Fig. 3 shows a second module 30 of the valve of the device 10 for maintaining PEEP, this second module 30 comprising a second connector 31 which materializes the second end 13 of the body of the valve 10 and being located on a first surface of a block 32 with a substantially cuboidal shape. In a second surface of the block 32, opposite to the first surface, a second connection member 33 is instead provided configured to connect to another module of the valve body, and preferably composed of a first section 34 with a parallelepiped shape with a square section, and a second section 35 with a parallelepiped shape with a rectangular section, directly connected to the block 32 and of the same width as the first section 34.

The connection member 33 comprises connection elements 36, for example holes configured to house rivets or screws, such holes being located in correspondence with the first section 34 and/or the second section 35, as shown in fig. 3.

Advantageously, the connection member 33, in particular its first section 34, is substantially coaxial to the second connector 31, so that it is possible to create a substantially rectilinear second part 11B of the internal duct 11 therein.

Two through apertures 37 can also be created in the block 32, in correspondence with a third and a fourth opposing surface, the through apertures 37 having a diameter similar or equal to the diameter of the second part 11B of the internal duct 11 and being suitable to receive possible accessories, such as pop-off valves and/or PEEP (Positive End-Expiratory Pressure) valves, for example. These through apertures 37 are oriented perpendicularly to the internal duct 11.

The valve comprises a third module 40, shown in fig. 4, inside which the closure member 14 is housed. The third module 40 comprises a lower element 41 and an upper element 42, both substantially U-shaped and reciprocally connected in such a way as to form a rectangular through aperture, inside which the closure member 14 is placed. The lower and upper elements 41, 42 have a length, taken in the direction of the internal duct 11, such as to be able to contain the connection members 23, 33 of the first and of the second module 20, 30, as well as the closure member 14 in all its positions.

In correspondence with a first side 40A of the rectangular aperture a first connection seating 43 is provided configured to connect to the connection member 23 of the first module 20, while on a second side 40B of the rectangular aperture a second connection seating 44 is provided configured to connect to the connection member 33 of the second module 30. These connection seatings 43, 44 are each provided with respective connection elements 45 positioned in such a way as to be aligned with the connection elements 24, 36 of the connection members 23, 33, respectively. In particular, the connection seatings 43, 44 and the connection members 23, 33 have corresponding shapes.

It is to be noted that, although the examples shown in the drawings provide a modular body divided into three modules, it can also be provided that the body, instead of being formed by the first module 20, the second module 30 and the third module 40, is made in a single piece. In this case, optimal levels of airtightness, ergonomics, compactness and lightness are guaranteed, and the length of the internal duct 11 is also substantially reduced, which allows to reduce the dead space, that is, the volume of air trapped in the circuit which does not participate in the diffusion of oxygen and carbon dioxide.

In accordance with a first variant, shown in figs. 1, 4 and 5, the valve is of the guillotine type. The closure member 14 comprises a plate 50 equipped with a retention element 51, for example a tooth, disposed on an upper edge 52 of the plate 50 so as to be able to retain the closure member in the opening position of the internal duct 11. A corresponding retention counter-member can be provided inside the third module 40, for example a pin, not shown in the drawings. This pin is advantageously part of the actuator 17, which in this variant can be of the solenoid actuator type.

Preferably, the plate 50 has a lower edge 53 the surface of which is inclined, for example by 45°, with respect to the faces of the plate 50 with a greater extension.

Advantageously, the sizes of the plate 50 are such as to completely close the internal duct 11 and it is inserted sliding in special grooves 46 inside the third module 40, the internal grooves 46 being located between the two connection seatings 43, 44 (fig. 4). In this variant, the opening position corresponds to a raised position of the guillotine closure member 14, while the closing position corresponds to a lowered position of the closure member 14. In this latter position, the plate 50 is flush with both connection members 23, 33 of the first and second module 20, 30 for a hermetic closure of the internal duct 11.

The displacement of the closure member 14 into the closing position, that is, in the lowered position, is preferably performed by means of thrust members, in particular springs, interposed between the upper edge 52 of the plate 50 and the upper part of the third module 40, after the pin no longer retains the retention element 51.

In one embodiment, another pin can be provided, or a similar mechanical or electro-mechanical member, which allows the operator to displace the guillotine manually in order to return it to the opening position, in which it is retained by the retention element 51.

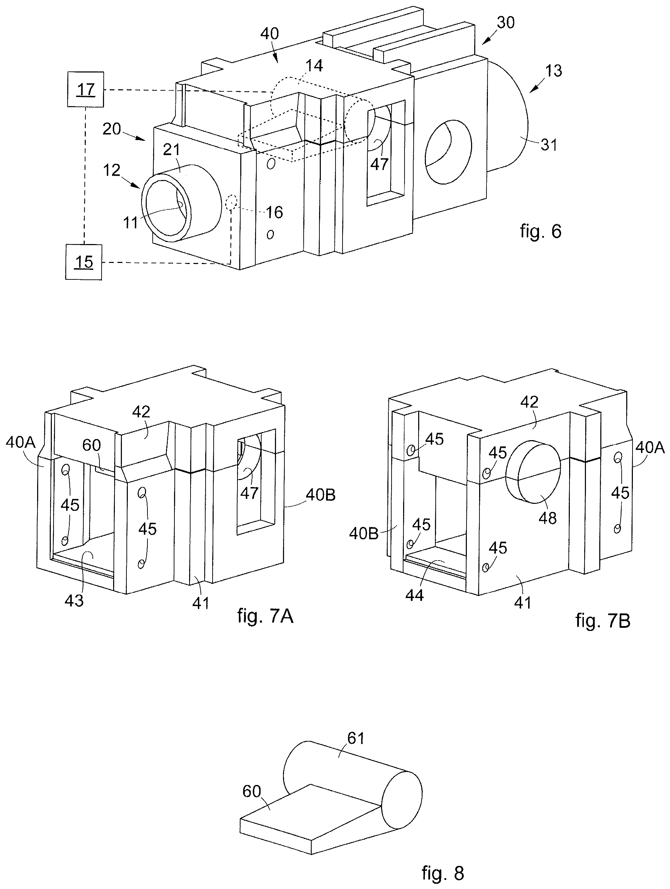

Fig. 6 shows a variant of the device 10 for maintaining PEEP, in which the first module 20 and the second module 30 of the valve are identical with respect to the first variant previously described.

This second variant differs from the first in the type of closure member 14, which in this variant is a trap door, that is, it comprises a flap 60 connected to a cylindrical rod 61 suitable to rotate around its own axis (fig. 8). The cylindrical rod 61 substantially coincides with an edge of the plate 60.

The third module 40 has a different shape compared to the first variant, in order to be able to house the closure member even when it is in the opening position, that is, with the flap substantially parallel to the direction of extension of the internal duct 11 (fig. 6). In its closing position, the flap of the closure member 14 is, instead, disposed in a substantially perpendicular position to the internal duct 11 so as to close it hermetically. For this purpose, in the closing position, the flap 60 is advantageously abutting against the connection member 33 of the second module 30, so as to hermetically close the second part 11B of the internal duct 11.

The third module is therefore more elongated than the first variant, and it comprises, on a first lateral wall, a hole 47 into which a first end of the cylindrical rod 61 is at least partly inserted (fig. 7A), and on a second lateral wall, opposite to the first one, an insertion seating 48 for the insertion of the other end of the cylindrical rod 61 (fig. 7B). The insertion seating 48 and the hole 47 are preferably coaxial so as to allow the correct rotation of the cylindrical rod 61.

In this variant, the actuator 17 can be of the servomotor type configured to make the trap door closure member 14 rotate.

In another version, the displacement of the trap door from the closing position to the opening position can be commanded through the actuator 17, which can possibly be commanded by the operator by means of a button disposed outside the valve body.

In both variants, it is possible to provide a signaling element, for example an LED which lights up when the closure member 14 is in the closing position.

In both variants, at least one pressure sensor 16 is preferably placed in the first module 20 (figs. 1 and 6) so as to monitor the pressure in the first part 11A of the internal duct 11, and thus detect a disconnection from the mechanical ventilation circuit 100 as soon as possible. It can be provided that, in the event the pressure drops below a predetermined threshold value, at least one pressure sensor 16 sends a corresponding signal to the controller 12 which commands, for example by means of the actuator 17, the displacement of the closure member 14 into the closing position of the internal duct 11.

Fig. 9 shows an example of a mechanical ventilation circuit 100 equipped with the device 10 for maintaining PEEP according to the present disclosure. The mechanical ventilation circuit 100 comprises a pulmonary ventilator 103, an inspiratory branch and an expiratory branch, both connected to the pulmonary ventilator 103. In the example given here, the inspiratory branch comprises a first gas delivery tube 105, a heated gas humidifier 104, suitably fed with sterile water, and a second gas delivery tube 106. The first tube 105 connects the pulmonary ventilator 103 to the humidifier 104, while the second tube leaves the humidifier 104 toward the patient. The expiratory branch is formed by a third tube 108 through which the gas exhaled by the patient returns toward the pulmonary ventilator 103. The inspiratory branch, in particular its second tube 106, and the expiratory branch both lead to a Y-shaped connector, identified by the reference number 107. The circuit also comprises a connection tube 102, interposed between the Y-shaped connector 107 and the artificial airway 101. The device 10 for maintaining PEEP is disposed between the artificial airway 101 inserted in the patient (endotracheal tube or tracheostomy tube) and the connection tube 102. More precisely, the device 10 is connected directly to the artificial airway 101 by means of the second end 13 of the body of the valve, and is connected directly to the connection tube 102 by means of the first end 12 of the body of the valve.

Modifications and/or additions of parts or steps may be made to the device for maintaining PEEP as described heretofore, without departing from the field and scope of the present invention, as defined by the claims.

Moreover, although the present invention has been described with reference to some specific examples, a person of skill in the art will be able to achieve other equivalent forms of device for maintaining the PEEP of a patient and mechanical ventilation circuit comprising such device, having the characteristics as set forth in the claims and hence all coming within the field of protection defined thereby.

In the following claims, the sole purpose of the references in brackets is to facilitate reading and they must not be considered as restrictive factors with regard to the field of protection defined by the same claims.

## Claims

1. Device (10) for maintaining PEEP for mechanical ventilation circuits (100), comprising
a valve equipped with a body that conforms an internal duct (11),
a closure member (14) which can be displaced between an opening position and
a closing position of said internal duct (11),
at least one pressure sensor (16) configured to monitor the pressure inside said internal duct (11) and
a controller (15) connected to said at least one pressure sensor (16) and to said closure member (14), configured to command the displacement of said closure member (14) to said closing position of said internal duct (11) when said at least one pressure sensor (16) detects a drop in pressure,
wherein said controller (15) is configured to command the displacement of said closure member (14) to said closing position of said internal duct (11) when said at least one pressure sensor (16) detects a drop in pressure below a predetermined threshold value, wherein said predetermined threshold value is preferably greater than, or equal, to the positive end-expiratory pressure (PEEP) and
wherein said body comprises a first end (12) configured to connect to a mechanical ventilation circuit (100) and a second end (13) configured to connect directly to an artificial airway (101) applicable to a patient,
said device being **characterized in that** it comprises an actuator (17) connected to said controller (15) and to said closure member (14), able to be commanded by said controller (15) to actuate the displacement of said closure member (14) and **in that** said closure member (14) is of the guillotine type.

2. Device as in claim 1, **characterized in that** said actuator (17) is a solenoidal actuator and comprises a pin inserted inside said valve and configured to retain said closure member (14) in the opening position, and **in that** it comprises one or more thrust members for thrusting said closure member (14) toward the closing position.

3. Device as in claim 2, **characterized in that** said closure member (14) comprises a plate (50) equipped, in correspondence with an upper edge (52) thereof, with a retention element (51) configured to be retained by said pin.

4. Device as in any claim hereinbefore, **characterized in that** said body is made in a single piece.

5. Mechanical ventilation circuit (100) comprising
a pulmonary ventilator (103),
an inspiratory branch (104, 105, 106) formed by one or more gas delivery tubes (105, 106) and on which there is disposed an active humidifier (104) fed with bi-distilled sterile water, and an expiratory branch (108),
a Y-shaped connector (107) which connects both said inspiratory branch and also said expiratory branch to a connection tube (102) connected to an artificial airway (101) which can be inserted into the natural airways of a patient, **characterized in that** it comprises
a device (10) for maintaining PEEP as in any claim hereinbefore interposed between said connection tube (102) and said artificial airway (101).

6. Mechanical ventilation circuit (100) as in claim 5, **characterized in that** said device (10) for maintaining PEEP is connected directly to said artificial connection tube (102).

## Patentansprüche

1. Gerät (10) zum Erhalten von PEEP für mechanische Beatmungskreisläufe (100), wobei dieses folgendes aufweist
ein Ventil, das mit einem Körper ausgestattet ist, der mit einem inneren Kanal (11) konform ist,
ein Schließelement (14), das zwischen einer offenen Stellung und einer geschlossenen Stellung des inneren Kanals (11) verschoben werden kann,
mindestens einen Drucksensor (16), der konfiguriert ist, den Druck innerhalb des inneren Kanals (11) zu überwachen und
ein Steuergerät (15), das mit dem mindestens einen Drucksensor (16) und mit dem Schließelement (14) verbunden ist, wobei dieses konfiguriert ist, die Verschiebung des Schließelements (14) zu der geschlossenen Stellung des inneren Kanals (11) anzuweisen, wenn der mindestens eine Drucksensor (16) einen Druckabfall ermittelt,
wobei das Steuergerät (15) konfiguriert ist, die Verschiebung des Schließelements (14) zu der geschlossenen Stellung des inneren Kanals (11) anzuweisen, wenn der mindestens eine Drucksensor (16) einen Druckabfall unter einen vorbestimmten Schwellenwert ermittelt, wobei der vorbestimmte Schwellenwert vorzugsweise größer als der oder gleich dem positive(n) endexspiratorische(n) Druck (PEEP) ist, und
wobei der Körper ein erstes Ende (12), das konfiguriert ist, sich mit einem mechanischen Beatmungskreislauf (100) zu verbinden, und ein zweites Ende (13) aufweist, das konfiguriert ist, sich direkt mit einem künstlichen Atemweg (101) zu verbinden, der an einem Patienten anwendbar ist,
wobei das Gerät **dadurch gekennzeichnet ist, dass**
es einen mit dem Steuergerät (15) und dem Schließelement (14) verbundenen Aktuator (17) aufweist, der in der Lage ist, durch das Steuergerät (15) angewiesen zu werden, die Verschiebung des Schließelements (14) in Gang zu bringen und **dadurch, dass** das Schließelement (14) Guillotinen-artig ist.

2. Gerät nach Anspruch 1, **dadurch gekennzeichnet, dass** der Aktuator (17) ein spulenförmiger Aktuator ist und einen in das Ventil eingeführten Stift aufweist, und konfiguriert ist, das Schließelement (14) in der offenen Stellung zu halten, und **dadurch, dass** es ein oder mehrere Schubelemente zum Schieben des Schließelements (14) in Richtung der geschlossenen Stellung aufweist.

3. Gerät nach Anspruch 2, **dadurch gekennzeichnet, dass** das Schließelement (14) eine Platte (50) aufweist, die, korrespondierend mit deren Oberrand (52), mit einem Halteglied (51) ausgestattet ist, das konfiguriert ist, durch den Stift gehalten zu werden.

4. Gerät nach einem der vorhergehenden Ansprüche, **dadurch gekennzeichnet, dass** der Körper einteilig hergestellt ist.

5. Mechanischer Beatmungskreislauf (100), der folgendes aufweist
ein Beatmungsgerät (103),
einen Einatemzweig (104, 105, 106), der durch einen oder mehrere Gaslieferschläuche (105, 106) gebildet ist, und an dem ein aktiver Befeuchter (104) angeordnet ist, der mit doppelt-destilliertem sterilem Wasser versorgt wird, und einen Ausatemzweig (108),
ein Y-förmiger Verbinder (107), der sowohl den Einatemzweig als auch den Ausatemzweig mit einem Verbindungsschlauch (102) verbindet, der mit einem künstlichen Atemweg (101) verbunden ist, der in den natürlichen Atemweg eines Patienten eingeführt werden kann, **dadurch gekennzeichnet, dass**
es ein Gerät (10) zum Erhalten von PEEP nach einem der vorhergehenden Ansprüche aufweist, das zwischen dem Verbindungsschlauch (102) und dem künstlichen Atemweg (101) angeordnet ist.

6. Mechanischer Beatmungskreislauf (100) nach Anspruch 5, **dadurch gekennzeichnet, dass**
das Gerät (10) zum Erhalten von PEEP direkt mit dem künstlichen Verbindungsschlauch (102) verbunden ist.

## Revendications

1. Dispositif (10) de maintien du PEEP pour circuits de ventilation mécanique (100), comprenant une soupape équipée d'un corps qui forme un conduit interne (11), un organe de fermeture (14) qui peut être déplacé entre une position d'ouverture et une position de fermeture dudit conduit interne (11), au moins un capteur de pression (16) configuré pour surveiller la pression à l'intérieur dudit conduit interne (11) et un contrôleur (15) connecté audit au moins un capteur de pression (16) et audit organe de fermeture (14), configuré pour commander le déplacement dudit organe de fermeture (14) vers ladite position de fermeture dudit conduit interne (11) lorsque ledit au moins un capteur de pression (16) détecte une chute de pression, lequel contrôleur (15) est configuré pour commander le déplacement dudit organe de fermeture (14) vers ladite position de fermeture dudit conduit interne (11) lorsque ledit au moins un capteur de pression (16) détecte une chute de pression en dessous d'une valeur seuil prédéterminée, lequel valeur seuil prédéterminée est de préférence supérieure ou égale à la pression expiratoire positive en fin d'expiration (PEEP) et lequel corps comprend une première extrémité (12) configurée pour être connectée à un circuit de ventilation mécanique (100) et une seconde extrémité (13) configurée pour être connectée directement à une voie aérienne artificielle (101) applicable à un patient, ledit dispositif étant **caractérisé en ce qu'**il comprend un actionneur (17) connecté audit contrôleur (15) et audit organe de fermeture (14), apte à être commandé par ledit contrôleur (15) pour actionner le déplacement dudit organe de fermeture (14) et **en ce que** ledit organe de fermeture (14) est de type guillotine.

2. Dispositif selon la revendication 1, **caractérisé en ce que** ledit actionneur (17) est un actionneur solénoïde et comprend une tige insérée à l'intérieur de ladite soupape et configurée pour retenir ledit organe de fermeture (14) en position d'ouverture, et **en ce qu'**il comprend un ou plusieurs organes de poussée pour pousser ledit organe de fermeture (14) vers la position de fermeture.

3. Dispositif selon la revendication 2, **caractérisé en ce que** ledit organe de fermeture (14) comprend une plaque (50) équipée, au niveau d'un bord supérieur (52) de celle-ci, d'un élément de retenue (51) configuré pour être retenu par ladite tige.

4. Dispositif selon l'une quelconque des revendications précédentes, **caractérisé en ce que** ledit corps est réalisé en pièce monobloc.

5. Circuit de ventilation mécanique (100) comprenant un ventilateur pulmonaire (103), une branche inspiratoire (104, 105, 106) formée par un ou plusieurs tubes d'acheminement de gaz (105, 106) et sur laquelle est disposé un humidificateur actif (104) alimenté en eau stérile bidistillée, et une branche expiratoire (108), un raccord en Y (107) qui connecte à la fois ladite branche inspiratoire et également ladite branche expiratoire à un tube de connexion (102) connecté à une voie aérienne artificielle (101) qui peut être insérée dans les voies aériennes naturelles d'un patient, **caractérisé en ce qu'**il comprend un dispositif (10) de maintien du PEEP selon l'une quelconque des revendications précédentes interposé entre ledit tube de connexion (102) et ladite voie aérienne artificielle (101).

6. Circuit de ventilation mécanique (100) selon la revendication 5, **caractérisé en ce que** ledit dispositif (10) de maintien du PEEP est connecté directement audit tube de connexion artificiel (102).
